# EUROPÄISCHE PATENTANMELDUNG

(11) **EP 3 295 898 A1**
(43) Veröffentlichungstag der Anmeldung: **21.03.2018**
(21) Anmeldenummer: 16189609.7
(22) Anmeldetag: 20.09.2016
(51) Int. Cl.: A61F 2/24, A61L 27/50, A61L 27/58, A61L 31/14

(54) **MEDIZINISCHES HYBRIDIMPLANTAT ZUM ABDICHTEN VON PARAVALVULÄREN LECKAGEN**

(71) Anmelder: Biotronik AG, 8180 Bülach (CH)
(72) Erfinder: Rothstock, Stephan, 18057 Rostock (DE); Forkmann, Christoph, 18057 Rostock (DE)
(74) Vertreter: Galander, Marcus

(57) **Zusammenfassung**

Die Erfindung beschreibt ein medizinisches Hybridimplantat zum Einführen mit einer Einführvorrichtung in den menschlichen oder tierischen Körper, insbesondere zum Abdichten von paravalvulären Leckagen sowie eine Einführvorrichtung zum Einführen eines medizinischen Hybridimplantats und ein Verfahren zum Einführen des medizinischen Hybridimplantats mit Hilfe der Einführvorrichtung.

## Beschreibung

Die Erfindung beschreibt ein medizinisches Hybridimplantat zum Einführen mit einer Einführvorrichtung in den menschlichen oder tierischen Körper, insbesondere zum Abdichten von paravalvulären Leckagen sowie eine Einführvorrichtung zum Einführen eines medizinischen Hybridimplantats und ein Verfahren zum Einführen des medizinischen Hybridimplantats mit Hilfe der Einführvorrichtung.

In der Medizin kommen häufig Implantate zum Einsatz, die zur Erfüllung von Ersatzfunktionen permanent oder zumindest für einen längeren Zeitraum in einen tierischen und/oder menschlichen Körper eingebracht werden. Zu nennen wären hier beispielsweise Herzschrittmacher, Hirnschrittmacher für Parkinsonpatienten, Herzimplantate, Cochleaimplantate, Retina Implantate, zahnmedizinische Implantate, Implantate zum Gelenkersatz, Gefäßprothesen oder Stents.

Die Verengung der Mitralklappe (Mitralklappenstenose) ist der häufigste Herzklappenfehler im hohen Lebensalter. Sie wird hervorgerufen durch eine Ablagerung von Kalziumsalzen (Kalzifizierung) an den Strukturen des Herzens und führt dort zu Verengungen. Durch diese Verengungen wird die Herzklappe daran gehindert, sich vollständig zu öffnen, wodurch der Fluss des Blutstroms vom Herzen in die Aorta be- oder gar verhindert wird. Klappenstenosen können zu lebensbedrohlichen Problemen führen, wie beispielsweise Herzversagen, Herzrhythmusstörungen, Herzstillstand oder Brustschmerzen. Auf dem Feld der Herzimplantate sind beispielsweise Klappenimplantate, wie etwa Aortenklappenimplantate, bekannt, welche die Funktion der natürlichen Aortenklappe übernehmen.

Bei der Transkatheter-Aortenklappen-Implantation (TAVI: Transcatheter Aortic Valve Implantation) handelt es sich um ein minimalinvasives Operationsverfahren, bei dem ein Klappenimplantat nach Expansion der Implantatstruktur sofort nach der Implantation im Herzen fixiert wird und die Position und Funktion der natürlichen Aortenklappe übernimmt. Trotz der unbestreitbaren Vorteile dieser Methodik kommt es innerhalb der ersten zwei Jahre nach der Implantation häufig zu paravalvulären Leckagen, welche durch eine lückenhafte Anpassung des Implantats an die Aortenwandung hervorgerufen wird. Grund hierfür ist die mehr oder weniger starke Kalzifizierung des Aortenannulus, welche durch eine der Transkatheter-Aortenklappen-Implantation vorgeschalteten Ballonexpansion nicht vollständig entfernt werden kann.

Es wurden bereits zahlreiche Anstrengungen unternommen, um Leckagen bei Transkatheter-Aortenklappen-Implantationen zu verhindern. Beispielsweise finden Hybridimplantate Anwendung, wobei vor der Implantation des Klappenimplantats sogenannte Abdichtringe in den Klappenbereich eingebracht werden. Diese Abdichtringe sind in der Regel wie ein Implantat aufgebaut, insbesondere wie ein Stent, wobei auf dem Implantatgerüst eine Dichtstruktur zur Lumenwand vorgesehen ist.

Beispielsweise wird in EP 2 789 313 ein Hybridimplantat beschrieben, wobei das erste Teilimplantat als ein Stent zum Abdichten von Leckagen ausgeführt ist, bei dem der Grundkörper aus einer Eisen-Legierung besteht, auf dessen Oberfläche eine Dichtstruktur aus einem Polyester, beispielsweise Dacron®, aufgebracht wird. Bei der Expansion des Implantats wird diese Dichtstruktur an die Lumenwand gedrückt, um so die Lumenwand vor Leckagen abzudichten. Das Abdichten erfolgt hier einerseits durch die polymere Dichtstruktur und andererseits durch die Radialkraft des Stents, welche das Grundgerüst des Implantats auf den Klappenbereich ausübt.

Allerdings weisen die bislang bekannten Systeme gleich mehrere Nachteile auf. Zum einen weisen die Dichtstrukturen ein ungenügendes Abdichtverhalten bei starker Verkalkung des Klappenöffnungsbereichs auf. Dieses Problem versucht der Fachmann in der Weise zu lösen, dass Implantate mit hoher Radialkraft, ca. 160 mmHg (Druck bezogen auf die Stentmantelfläche), eingesetzt werden, wodurch die Dichtstruktur fest an die Außenwand gedrückt wird. Allerdings können auch auf diese Weise Leckagen nicht gänzlich vermieden werden, da starke Verkalkungen auf diese Weise nicht gänzlich abgedichtet werden können. Zudem wirkt sich eine zu hohe Radialkraft nachteilig auf den Annulus aus, da hierdurch das Reizleitungssystem im Herzen wesentlich irritiert werden kann.

Zum anderen besteht der Grundköper des Abdichtrings aus einem nicht biodegradierbaren oder langsam biodegradierbaren Material, wodurch weitere Nachteile entstehen. Belässt man ein nicht biodegradierbares oder langsam biodegradierbares Implantat nach erfolgreicher Therapie im Körper, so besteht die Gefahr einer dauerhaften Reizung des umliegenden Gewebes. Weiterhin bleibt durch die natürliche Bewegung innerhalb des Klappenöff nungsbereiches der Abdichtring selbst in gewissem Maße beweglich, so dass Leckagen jederzeit wieder auftreten können. Zudem werden eine Vielzahl der Abbauprodukte der langsam biodegradierbaren Implantate vom Körper nicht verstoffwechselt. Restenosen (Wiederverengung des Gefäßes) und Thrombosen sind daher nie gänzlich zu vermeiden. Weiterhin führt ein dauerhafter Kontakt zwischen dem Abdichtring und dem später eingesetzten Klappenimplantat zu unerwünschten Abriebsphänomenen. Unter anderem kann es zur Ausbildung von Lokalelementen kommen, die durch den Kontakt von Metallen unterschiedlicher elektrochemischer Spannung zu Stande kommt. Diese Lokalelemente beeinflussen wesentlich die Materialeigenschaften der Implantate, welche nicht immer vorhersehbar sind.

Es ist deshalb Aufgabe der vorliegenden Erfindung, ein Hybridimplantat bereitzustellen:
- welches paravalvuläre Leckagen verhindert,
- welches ausreichende mechanische Eigenschaften besitzt, um eine Dichtstruktur an die Gefäßwand zu bringen ohne diese dabei zu stark zu weiten, und
- welches einen dauerhaften Kontakt der einzelnen Teilimplantate des Hybridimplantats verhindert.

Eine weitere Aufgabe ist in der Bereitstellung einer Einführvorrichtung zu sehen, welche das Freigeben eines Hybridimplantats ermöglicht.

Eine weitere Aufgabe kann darin gesehen werden, einen Abdichtring zum Aufnehmen eines mit einem Klappeneinsatz versehenen Teilimplantats bereitzustellen.

Eine zudem weitere Aufgabe kann darin gesehen werden, ein Verfahren zum Einführen eines erfindungsgemäßen medizinischen Hybridimplantats mit Hilfe der erfindungsgemäßen Einführvorrichtung bereitzustellen.

Die Aufgabe wird erfindungsgemäß durch die Merkmale der unabhängigen Ansprüche gelöst. Günstige Ausgestaltungen und Vorteile der Erfindung ergeben sich aus den weiteren Ansprüchen und der Beschreibung.

Es wird ein medizinisches Hybridimplantat zum Einführen in den menschlichen oder tierischen Körper vorgeschlagen, wobei das Hybridimplantat zumindest zwei Teilimplantate aufweist, von denen das zumindest erste Teilimplantat fremdexpandierbar ausgeführt wird sowie aus einem mit einer quellfähigen biodegradierbaren Polymermatrix versehenen biokorrodierbaren Material besteht und das zumindest zweite Teilimplantat fremdexpandierbar oder selbstexpandierbar ausgeführt wird sowie mit einem Klappenersatz versehen ist.

Die vorliegende Erfindung beruht auf der Erkenntnis, dass durch die Verwendung eines zumindest ersten Teilimplantats, bestehend aus einem biokorrodierbaren Material, welches mit einer quellfähigen biodegradierbaren Polymermatrix versehen ist, ein Hybridimplantat bereitgestellt wird, das eine oder mehrere der vorstehenden Aufgaben löst. Durch das erfindungsgemäße Hybridimplantat kann ein Abdichtsystem für Klappenöffnungen bereitgestellt werden, welches zu einer besseren Konformität zwischen dem Klappenimplantat und den Gefäßwänden an den Klappenöffnungen führt. Bedingt durch die quellfähige biodegradierbare Polymermatrix des zumindest ersten Teilimplantats werden die Deformität und/oder die Verkalkungen des Klappenbereichs überdeckt. Bedingt durch seine chemischen, physikalischen und/oder mechanischen Eigenschaften schließt das quellfähige Material kleinste Lücken, welche zwischen dem Klappenbereich und/oder Gefäßwand und dem Implantat auftreten und verhindert so paravalvuläre Leckagen. Erfindungsgemäß dichtet das zumindest erste Teilimplantat den Klappenbereich durch in Kontakt bringen und/oder halten der quellfähigen biodegradierbaren Polymermatrix mit dem Klappenbereich ab, wodurch vorteilhafterweise eine deutlich geringe Radialkraft aufgebracht werden muss, welches die Reizung dieses anatomisches Bereiches deutlich reduziert. Weiterhin vorteilhaft kann mittels der erfindungsgemäßen Ausgestaltungen die Metallmenge, welche im Körper dauerhaft verbleibt, deutlich reduziert werden, was besonders patientenfreundlich ist. Durch den Einsatz eines zumindest ersten Teilimplantats, bestehend aus einem biokorrodierbaren Material, können unerwünschte Abriebphänomene sowie weitere Reizungen bedingt durch eine dauerhafte Kontaktierung zwischen dem Abdichtring und dem Klappenimplantat deutlich reduziert oder gar verhindert werden. Zudem werden eine Vielzahl der Abbauprodukte des biokorrodierbaren Teilimplantats vom Körper verstoffwechselt, wodurch das Auftreten von Restenosen und Thrombosen ebenfalls deutlich reduziert wird.

Unter "fremdexpandierbar" soll im Rahmen der vorliegenden Erfindung "passiv expandierbar" und/oder, dass das Teilimplantat unselbstständig und/oder mittels einer von außen zugeführten Kraft expandierbar bzw. plastisch verformbar ist, verstanden werden. Konstruktiv einfach kann die passive Expansion mittels einer Expansionshilfe, wie beispielsweise eines mechanischen, pneumatischen und/oder hydraulischen Spreizers oder, vorteilhaft, eines Ballons, erfolgen. Unter "selbstexpandierbar" soll im Rahmen der vorliegenden Erfindung "aktiv expandierbar" und/oder dass das Teilimplantat selbstständig bzw. selbsttätig, also ohne fremden Hilfe, expandiert bzw. expandierbar ist, verstanden werden. Dem Fachmann sind die unterschiedlichen Methoden zur Expansion von Implantaten bekannt.

Gemäß der vorliegenden Erfindung soll unter einem medizinischen Hybridimplantat ein Implantat aus zumindest zwei Teilimplantaten verstanden werden, wobei die Teilimplantate räumlich und/oder funktional voneinander unabhängig ausgeführt sind. Bevorzugt setzt sich das Hybridimplantat aus zwei separaten Teilimplantaten zusammen. Hierbei stellt ein Teilimplantat ein in sich eigenständig funktionierendes und/oder einsetzbares Bauteil dar.

In diesem Zusammenhang soll unter einem "Teilimplantat" oder "Implantat" insbesondere ein Körper verstanden werden, der zumindest eine Ersatzfunktion permanent oder für einen gewissen Zeitraum bei Implantation in einen tierischen oder menschlichen Körper erfüllt. Denkbar wären hier sämtliche, dem Fachmann als zweckdienlich erscheinende medizinische Implantate, wie beispielsweise ein Herzimplantat oder eine Gefäßprothese. Besonders vorteilhaft wird die Ausbildung des medizinischen Hybridimplantats als ein Klappenimplantat vorgeschlagen. Unter einem "Klappenimplantat" soll insbesondere ein Körper verstanden werden, der zumindest eine Ersatzfunktion eines Rückschlagventils, permanent oder für einen längeren Zeitraum bei Implantation erfüllt. Denkbar wären hier sämtliche, dem Fachmann als zweckdienlich erscheinende medizinische Klappenimplantate, wie beispielsweise ein Aortenklappen-, ein Pulmonalklappen-, ein Mitralklappen- oder ein Trikuspidalklappenimplantat, eventuell auch Venenklappen.

Gemäß der vorliegenden Erfindung besteht das zumindest erste Teilimplantat aus einem biokorrodierbaren Material. Im Sinne der vorliegenden Erfindung bedeutet der Begriff "biokorrodierbares Material", dass in physiologischer Umgebung ein Abbau/LTmbau des Werkstoffs, aus dem das Teilimplantat besteht, stattfindet, so dass der aus dem Werkstoff bestehende Teil des Implantates ganz oder zumindest überwiegend nicht mehr vorhanden ist. Vorzugsweise besteht der Werkstoff des Implantats aus einer biokorrodierbaren Magnesiumlegierung. Unter Magnesiumlegierung wird hier ein metallisches Gefüge verstanden, dessen Hauptkomponente Magnesium ist. Hauptkomponente ist die Legierungskomponente, deren Gewichtsanteil an der Legierung am höchsten ist. Ein Anteil der Hauptkomponente beträgt vorzugsweise mehr als 50 Gew.%, insbesondere mehr als 70 Gew.%, ganz besonders bevorzugt mehr als 90 Gew.%. Vorzugsweise enthält die biokorrodierbare Magnesiumlegierung des Kerns Yttrium und weitere Seltenerdmetalle, da sich eine derartige Legierung aufgrund ihrer physiko-chemischen Eigenschaften und hohen Biokompatibilität, insbesondere auch seiner Abbauprodukte, auszeichnet. Unter der Sammelbezeichnung "Seltenerdmetalle" werden vorliegend Scandium (21), Yttrium (39), Lanthan (57) und die 14 auf Lanthan (57) folgenden Elemente, nämlich Cer (58), Praseodym (59), Neodym (60), Promethium (61), Samarium (62), Europium (63), Gadolinium (64), Terbium (65), Dysprosium (66), Holmium (67), Erbium (68), Thulium (69), Ytterbium (70) und Lutetium (71) verstanden. Dem Fachmann ist eine Vielzahl von Magnesiumlegierungen bekannt. Besonders bevorzugt ist eine Magnesiumlegierung der Zusammensetzung Seltenerdmetalle 5,2 - 9,9 Gew.%, davon Yttrium 3,7 - 5,5 Gew.%, und Rest < 1 Gew.%, wobei Magnesium den auf 100 Gew.% fehlenden Anteil an der Legierung einnimmt. Insbesondere bevorzugt ist eine Magnesiumlegierung der Zusammensetzung Yttrium 3,7 - 5,5 Gew.%, Neodym 1,5 - 3 Gew.%, Zirkonium 0,3 - 1 Gew.%, Gadolinium 0,4 - 0,55 Gew.%, Dysprosium 0,4 - 0,55 Gew.%, Eisen < 0,006 und Rest < 1 Gew.%, wobei Magnesium den auf 100 Gew.% fehlenden Anteil an der Legierung einnimmt. Weiterhin bevorzugt sind Legierungen auf Basis von Magnesium der Form Mg-Zn-Al, Mg-Zn-Ca, Mg-Zn oder Mg-Al, wobei diese Legierungen höchstens Spuren von Seltenerdmetalle oder weiteren Elementen aufweisen. Bevorzugt ist eine Magnesiumlegierung der Zusammensetzung Zink 1,5 - 7,0 Gew.%, Aluminium 0,5 - 10,0 Gew.%, Rest < 0,0063 Gew.%, wobei Magnesium den auf 100 Gew.% fehlenden Anteil an der Legierung einnimmt. Weiterhin bevorzugt ist eine Magnesiumlegierung der Zusammensetzung Zink 3,0 - 7,0 Gew.%, Kalzium 0,001 - 0,6 Gew.%, Rest < 0,0048 Gew.%, wobei Magnesium den auf 100 Gew.% fehlenden Anteil an der Legierung einnimmt. Diese Magnesiumlegierungen bestätigten ihre besondere Eignung, d. h. zeigt eine hohe Biokompatibilität, günstige Verarbeitungseigenschaften, gute mechanische Kennwerte und ein für die Einsatzzwecke adäquates Korrosionsverhalten.

Das aus einem biokorrodierbaren Material bestehende zumindest erste Teilimplantat weist erfindungsgemäß eine quellfähige biodegradierbare Polymermatrix auf. In diesem Zusammenhang soll unter einer "biodegradierbaren Polymermatrix" ein Polymer verstanden werden, welches die Fähigkeit hat, sich in physiologischer Umgebung kontrolliert und vornehmlich ohne Irritation des sich umgebenden Organismus aufzulösen. Erfindungsgemäß soll die Polymermatrix quellfähig sein, d. h. die Eigenschaft besitzen, in physiologischer Umgebung durch einen externen Stimulus seine Oberfläche derart zu verändern, dass es zu einer Volumenvergrößerung kommt. Dieser Stimulus kann unterschiedlicher Natur sein, beispielsweise die Temperatur, die Feuchtigkeit, der pH, das Licht, ein elektrisches oder magnetisches Feld. Dem Fachmann sind diese chemischen, physikalischen und/oder biologischen Stimuli bekannt. Besonders bevorzugt reagiert die quellfähige Polymermatrix auf Körperflüssigkeiten wie Wasser oder Blut. Bevorzugt quillt die erfindungsgemäße Polymermatrix um den Faktor 100, besonders bevorzugt um den Faktor 50 bis 90, insbesondere um den Faktor 60. Dabei dringen Blut und/oder andere Körperflüssigkeiten in die Polymermatrix ein, absorbiert diese und quillt derart, dass es zur gewünschten Volumenvergrößerung kommt. Bevorzugt handelt es sich bei der Polymermatrix um ein Hydrogel.

Ein Hydrogel ist ein wasserenthaltendes, aber wasserunlösliches Polymer, dessen Moleküle chemisch, z.B. durch kovalente oder ionische Bindungen oder physikalisch, z.B. durch Verschlaufen der Polymerketten, zu einem dreidimensionalen Netzwerk verknüpft sind. Erfindungsgemäße Hydrogele sind in der Lage ihr Volumen zu verändern, in dem sie bei Kontakt mit Körperflüssigkeiten quellen. Diese Hydrogele können beispielsweise hergestellt werden durch Umsetzung ethylenisch ungesättigter Monomere und Polymere die ionisierbare Gruppen tragen mit Vernetzern und Polymerisationskatalysatoren. Alternativ dazu können geeignete Hydrogele hergestellt werden durch Kondensationsreaktionen mit difunktionellen und mehrfunktionellen Monomeren. Bevorzugt sind die Hydrogele ausgewählt aus der Gruppe umfassend Hyaluronsäuren, Polysaccharide, Polyphosphazene, Cellulose, Polyehtylenglycole, oder Polyacrylamid einzeln oder in Kombination.

Erfindungsgemäß wird die quellfähige Polymermatrix gemäß dem Fachmann bekannter Verfahren auf das zumindest erste Teilimplantat aufgebracht. Denkbar sind hier Aufsprühverfahren, Tauchverfahren oder Spinnverfahren.

In einer bevorzugten Ausgestaltung beträgt die Radialkraft des zumindest ersten Teilimplantats 1/2, insbesondere 1/3, ganz besonders 1/4 der Radialkraft des zumindest zweiten Teilimplantats. Die Radialkraft des zumindest zweiten Teilimplantats beträgt erfindungsgemäß zwischen 120 und 180 mmHg, bevorzugt 160 mmHg. In einer besonderen Ausgestaltung der Erfindung beträgt die Radialkraft des zumindest ersten Teilimplantats zwischen 30 bis 80mmHg.

Das erfindungsgemäße zumindest erste Teilimplantat dichtet den Klappenbereich durch in Kontakt bringen und/oder halten der erfindungsgemäßen quellfähigen biodegradierbaren Polymermatrix mit dem Klappenbereich ab. Eine Stützfunktion des Klappenbereichs oder ein Offenhalten der Klappen ist erfindungsgemäß nicht zwingend notwendig. Erfindungsgemäß reicht eine Radialkraft des zumindest ersten Teilimplantats aus, die verhindert, dass das Implantat durch die Fließkraft des Blutstroms weggespült wird. Durch die polymere Matrix erfolgt durch den Formschluss des quellfähigen Polymers einerseits und zusätzlich durch die geringe Radialkraft des Teilimplantats andererseits eine Haltekraft am Klappenbereich, die ausreichend ist, um das Abdichtsystem so lange am gewünschten Implantationsort zu halten, bis das zumindest zweite Teilimplantat implantiert wird.

Erfindungsgemäß wird ein Abdichtsystem bereitgestellt, welches eine dauerhafte Abdichtfunktion des Hybridimplantats ermöglicht. In den ersten Stunden bis hin zu einigen Wochen nach der Implantation übernimmt das erfindungsgemäße zumindest erste Teilimplantat durch die Eigenschaften des Implantatmaterials und/oder der quellfähigen biodegradierbaren Polymermatrix eine erste Abdichtfunktion. Während der Degradation und Korrosion der einzelnen Komponenten des zumindest ersten Teilimplantats verbleibt nach vollständigem Abbau lediglich ein abdichtender Thrombus, welcher den Klappenbereich langfristig abdichtet.

In einer besonderen Ausgestaltung des zumindest ersten Teilimplantats kann ein Formschluss zwischen dem Annulus des Klappenöffnungsbereichs und dem Teilimplantat erfolgen.

Gemäß der vorliegenden Erfindung wird das zumindest zweite Teilimplantat fremdexpandierbar oder selbstexpandierbar, bevorzugt selbstexpandierbar ausgeführt. Das zumindest zweite selbstexpandierbare Teilimplantat besteht aus einem elastischen oder superelastischen Material, etwa einem metallischen Material und/oder aus einer Kombination von mehreren metallischen Materialien, wie beispielsweise Eisen, Magnesium, Nickel, Wolfram, Titan, Zirkonium, Niob, Tantal, Zink, Silizium, Lithium, Natrium, Kalium, Kalzium, Mangan und/oder jedem anderen, dem Fachmann als sinnvoll erscheinendem Material. Vorteilhafterweise ist das zumindest zweite selbstexpandierbare Teilimplantat aus einem Formgedächtnis-Material, wie beispielsweise einer Kupfer-Zink-Aluminium-Legierung und/oder Nickel-Titan-Legierung, vorzugsweise Nitinol, gefertigt.

Das zumindest zweite Teilimplantat umfasst erfindungsgemäß zumindest einen Klappeneinsatz, wodurch das Hybridimplantat eine Ersatzfunktion, insbesondere als Rückschlagventil, im Körper übernehmen kann. Vorteilhafterweise kann sich das selbstexpandierbare Teilimplantat flexibel an die Patientenanatomie anpassen, wodurch sich auch der Klappeneinsatz vorteilhaft an die Gegebenheiten anpassen kann. Hierbei kann der Klappeneinsatz mittels jeder, dem Fachmann für sinnvoll erachteter Verbindungsart, wie beispielsweise Nähen und/oder Kleben, mit dem selbstexpandierbaren Teilimplantat verbunden sein. In diesem Zusammenhang soll unter "Klappeneinsatz" insbesondere eine Aortenklappe, eine Pulmonalklappe, eine Mitralklappe, eine Trikuspidalklappe oder eine Venenklappe aus natürlichem und/oder künstlichem Material verstanden werden. Besonders bevorzugt ist eine Ausgestaltung des medizinischen Hybridimplantats als Mitralklappe vorgesehen, wodurch eine Ersatzstruktur für die am häufigsten mit Fehlfunktionen behaftete Herzklappe bereitgestellt werden kann. Ebenso ist eine Ausgestaltung als Aortenklappe oder Pulmonalklappe denkbar.

Das Klappenmaterial kann künstlichen oder natürlichen Ursprungs (Rind, Schwein, Pferd) sein. Das Klappenmaterial kann beispielsweise aus künstlichem polymerem Material gebildet sein oder ein solches umfassen. In einer bevorzugten Ausführungsform ist das Klappenmaterial aus Polyester, Polyolefin, insbesondere Polyethylen, Polypropylen und Polytetrafluoroethylen, oder Polyester, insbesondere Dacron® ausgebildet. Das Klappenmaterial kann auch eines der folgenden Materialen aufweisen oder aus einem der folgenden Materialien bestehen: mikrobielle Cellulose, oder ferner grundsätzlich alle Arten von tierischem Gewebe, insbesondere Gewebe vom Säugetier, einschließlich vom Menschen. Bevorzugt ist nicht-humanes Gewebe. Insbesondere eignen sich solche Gewebe, die als Klappenmaterial in einer Herzklappe verwendet werden können. Bevorzugt sind dabei Perikardgewebe und Herzklappen, aber auch Haut, Bandgewebe, Sehnen, Bauchfell, Dura mater, Tela submucosa, insbesondere des Magen-Darm-Traktes, oder Rippenfell. Bei Herzklappen können alle Klappen verwendet werden, also Aorten-, Pulmonal-, Mitral- und Trikuspidalklappen. Des Weiteren sind bevorzugt Perikardgewebe vom Schwein, Schaf, Ziege, Pferd, Krokodil, Känguru, Strauß und vom Rind.

Bevorzugt ist die Klappe des Klappeneinsatzes aus Rinder- oder Schweineperikard gefertigt.

Des Weiteren kann es vorteilhaft sein, wenn das medizinische Hybridimplantat ein Trennmittel aufweist, welches das Material des ersten fremdexpandierbaren Teilimplantats von dem Material des zweiten Teilimplantats bzw. deren Interaktionsbereich trennt. In diesem Zusammenhang soll unter einem "Trennmittel" insbesondere jedes, dem Fachmann für zweckdienlich erscheinendes Mittel, wie Abstandshalter und/oder insbesondere eine Beschichtung verstanden werden. Unter einer "Beschichtung" soll eine zumindest teilweise und bevorzugt eine vollständige Ummantelung der Interaktionsbereiche bzw. deren Verstrebungen bzw. Stentstruts verstanden werden. Besonders vorteilhaft ist die Beschichtung von einem halbleitenden, isolierenden und passivierenden Siliziumcarbid gebildet und auf dem zumindest zweiten Teilimplantat vorgesehen. Generell wäre jedoch jede andere, dem Fachmann für einsetzbar erscheinende Beschichtung denkbar, die ein Inkontaktbringen der Materialien der beiden Teilimplantate in Gegenwart von Elektrolyten wirkungsvoll verhindert. Durch die Beschichtung kann konstruktiv einfach und Platz sparend ein Kontaktproblem der unterschiedlichen edlen Metalle der beiden Bereiche gelöst werden, so dass die Bildung von Lokalelementen und die daraus folgenden Nachteile wie beispielsweise hohe Zersetzungsrate der Magnesiumlegierung, verhindert werden kann.

Gemäß einer weiteren vorteilhaften Ausgestaltung weisen das zumindest erste Teilimplantat und das zumindest zweite Teilimplantat eine Anzahl von Zellen auf. Diese Zellen können jede, dem Fachmann als zweckdienlich erscheinende Form, wie rund, oval, dreieckig, rechteckig und/oder Rautenform aufweisen. Diese Form ist insbesondere dazu ausgebildet, um faltbar zu sein. Besonders bevorzugt sind die Zellen im Wesentlichen rautenförmig ausgestaltet. Unter der Wendung "im Wesentlichen rautenförmig" soll hier verstanden werden, dass auch Formen, die einer Raute bzw. einem Rhombus ähnlich sind, wie beispielsweise eine rautenähnliche Form mit abgerundeten Ecken und/oder konkaven und/oder konvexen Seiten unter dem Begriff "rautenförmig" verstanden werden. Die Zellen stellen das Gerüst des Teilimplantats dar. Ferner soll in diesem Zusammenhang unter dem Begriff "Grundgerüst" insbesondere eine Struktur, wie beispielsweise ein Drahtgeflecht, verstanden werden, die im Wesentlichen eine Gestalt und/oder eine Form des Teilimplantats vorgibt. Günstigerweise ist die Anzahl der Zellen so ausgewählt, dass darüber die Radialkraft des Teilimplantats so steuerbar ist, sprich dass die Radialkraft des zumindest ersten Teilimplantats 1/2, insbesondere 1/3, ganz besonders 1/4 der Radialkraft des zumindest zweiten Teilimplantats beträgt. Zudem kann über die Anzahl der Zellen der Crimpdurchmesser des Hybridimplantats beeinflusst werden. Je geringer der Crimpdurchmesser ausfällt, desto mehr Bauraum fürs Hybridimplantat kann eingespart werden, wodurch eine patientenfreundliche Einführvorrichtung mit insbesondere geringen French-Größen zum Einsatz kommt. In einer weiteren vorteilhaften Ausgestaltung weisen das zumindest erste Teilimplantat und das zumindest zweite Teilimplantat eine Anzahl von Zellen auf, welche mäanderförmig das Grundgerüst ausbilden. Dieses Design ist dem Fachmann bekannt.

In einer besonders bevorzugten Ausgestaltung des zumindest ersten Teilimplantats weist das Grundgerüst Bereiche unterschiedlicher Ausgestaltung auf. Beispielsweise kann das Grundgerüst einen festeren Mittelbereich und einen flexiblen Randbereich aufweisen oder umgekehrt. Andererseits kann das Grundgerüst auch unterschiedliche Wandstärken oder Strutbreiten aufweisen. Mit Hilfe dieser Ausgestaltungen kann man in vorteilhafter Weise die Radialkraft und die Formschlusseigenschaften zwischen dem Abdichtring und dem Annulus steuern, um so Leckagen verhindert.

Gemäß einem weiteren Aspekt der Erfindung wird eine Einführvorrichtung zum Abdichten von paravalvulären Leckagen vorgeschlagen, insbesondere zum Einführen eines eingangs beschriebenen medizinischen Hybridimplantats, wobei das Hybridimplantat zumindest zwei Teilimplantate aufweist, von denen zumindest ein erstes Teilimplantat fremdexpandierbar und zumindest ein zweites Teilimplantat fremdexpandierbar oder bevorzugt selbstexpandierend ausgeführt ist.

Erfindungsgemäß sind Einführvorrichtungen denkbar, die geeignet sind, ein eingangs beschriebenes medizinisches Hybridimplantat an seinen Bestimmungsort einzubringen und so paravalvulären Leckagen abzudichten. Dabei können das zumindest erste Teilimplantat mit einer separaten Einführvorrichtung implantiert werden als das zumindest zweite Teilimplantat. In einer besonderen Ausgestaltung der erfindungsgemäßen Einführvorrichtung wird das medizinische Hybridimplantat mittels einer Einführvorrichtung in den Körper eingeführt.

Bevorzugt weist die Einführvorrichtung, beispielsweise ein Katheter, ein erstes Einführelement, das zumindest eine Expansionshilfe zum Expandieren des zumindest ersten fremdexpandierbaren Teilimplantats umfasst, und ein zweites Einführelement zur Freigabe des zumindest zweiten selbstexpandierenden Teilimplantats. Dem Fachmann sind geeignete Methoden zum Freisetzen von fremdexpandierbaren und/oder selbstexpandierbaren Implantaten bekannt.

Durch die erfindungsgemäße Ausgestaltung kann eine Einführvorrichtung, beispielsweise ein Katheter, bereitgestellt werden, mit der das erfindungsgemäße Hybridimplantat besonders schnell und zuverlässig positioniert werden kann. Ebenso kann eine sichere Verankerung des Hybridimplantats am Implantationsort, wie einem Annulus, bereitgestellt werden, welche sich zusätzlich dadurch auszeichnet, dass paravalvuläre Leckagen verhindert werden. Zudem kann mit solch einer Einführvorrichtung ein kompaktes System verwendet werden, da eine radiale Lagerung der Bestandteile der Einführvorrichtung möglich ist und dadurch eine Länge der Einführvorrichtung gegenüber Systemen des Standes der Technik verkürzt werden kann. Des Weiteren können Vorteile von Systemen mit fremdexpandierbaren und selbstexpandierenden Implantaten in einer Einführvorrichtung vereint werden. Dies wäre z.B. eine gute Anpassbarkeit des selbstexpandierenden Teilimplantats an eine Patientenanatomie am Implantationsort, wie beispielsweise einem Annulus einer nativen Herzklappe. Ebenso ist eine stabile Befestigung und Abdichtung mit Hilfe des fremdexpandierbaren ersten Teilimplantats am Implantationsort möglich.

Weiterhin wird ein implantierbarer Abdichtring zum Aufnehmen eines mit einem Klappeneinsatz versehenen Teilimplantats vorgeschlagen, welcher die oben dargelegten Merkmale des zumindest ersten Teilimplantats aufweist. Um Wiederholungen vorzubeugen, wird auf die oben genannten Vorteile des zumindest ersten Teilimplantats verwiesen.

Der erfindungsgemäße Abdichtring kann in einer besonderen Ausgestaltung in axialer Richtung sehr kurz ausgeführt werden, in der Weise, dass lediglich der Bereich des Annulus abgedeckt wird. Somit wird einerseits die eigentliche Funktion der Klappen selbst nicht beeinträchtigt und andererseits ein gezieltes Abdichten des Annulus ermöglicht.

Zudem wird ein Verfahren zum Einführen eines medizinischen Hybridimplantats mit Hilfe einer Einführvorrichtung vorgeschlagen.

Dabei kann in einem ersten Schritt das vollständige Expandieren des zumindest ersten Teilimplantats mittels entsprechender Einführvorrichtung erfolgen, gefolgt von der Freisetzung im zweiten Schritt des zumindest zweiten, bevorzugt selbstexpandieren, Teilimplantats. Die Maßnahmen zum jeweiligen Freisetzen der unterschiedlichen Teilimplantate sind dem Fachmann bekannt.

Durch das erfindungsgemäße Verfahren kann das Hybridimplantat besonders schnell und zuverlässig positioniert, verankert sowie implantiert werden, in der Weise, dass paravalvuläre Leckagen verhindert werden. Des Weiteren können mit den beiden ungleich expandierbaren Teilimplantaten vorteilhaft unterschiedliche Expansionsmechanismen angewendet werden, was zu einem besonders vielseitig einsetzbaren Verfahren führt. Dadurch können Vorteile von beiden Expansionsmechanismen in einem Verfahren vereint werden.

Die Erfindung ist nachfolgend beispielhaft, anhand von einem in Zeichnungen dargestellten Ausführungsbeispiel, näher erläutert. Es zeigen in schematischer Darstellung:
- Fig. 1: das erstes fremdexpandierbares Teilimplantat mit einer schematisch gezeichneten quellfähigen biodegradierbaren Polymermatrix;
- Fig. 2: ein zweites bevorzugt selbstexpandierendes Teilimplantat des Hybridimplantats in einer Detaildarstellung;
- Fig. 3: eine bevorzugte Geometrie eines ersten fremdexpandierbaren Teilimplantats in einer Detaildarstellung
- Fig. 4: eine schematische Darstellung einer Implantation eines zumindest ersten Teilimplantats/Abdichtrings in den Annulus des Klappenöffnungsbereichs

In den Figuren sind funktionell gleiche oder gleich wirkende Elemente jeweils mit denselben Bezugszeichen beziffert. Die Figuren sind schematische Darstellungen der Erfindung. Sie bilden nicht spezifische Parameter der Erfindung ab. Weiterhin geben die Figuren lediglich typischen Ausgestaltungen der Erfindung wieder und sollen die Erfindung nicht auf die dargestellten Ausgestaltungen beschränken.

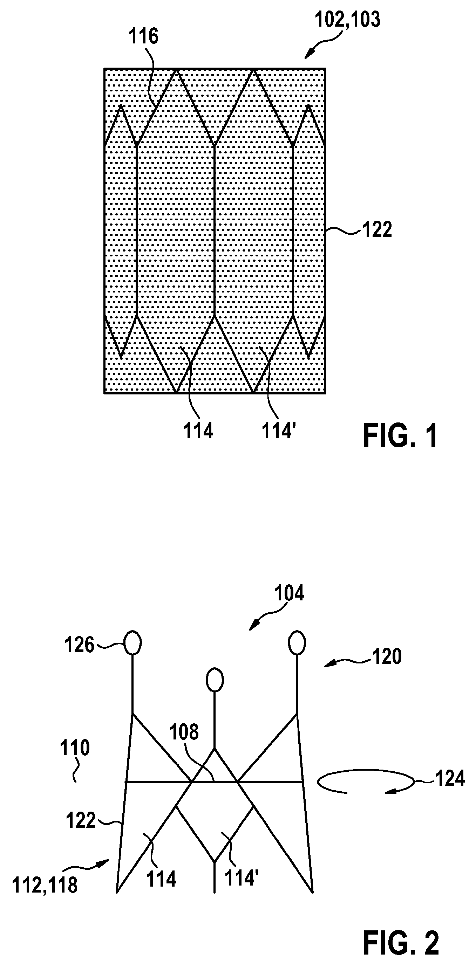

Wie in der Fig. 1 gezeigt ist, weist das fremdexpandierbare Teilimplantat 102 oder der Abdichtring 103 eine quellfähige biokorrodierbare Polymermatrix 116 auf. Diese ist dazu ausgebildet, im implantierten Zustand eine, insbesondere valvuläre und/oder eine paravalvuläre Leckage zu reduzieren. Die quellfähige biokorrodierbare Polymermatrix 116 kann beispielsweise aus einem Hydrogel gebildet sein. Zudem weist das fremdexpandierende Teilimplantat 102 ein Grundgerüst 122 mit einer Anzahl von Zellen 114, 114' auf. Diese sind erfindungsgemäß so ausgestaltet, dass die Radialkraft des zumindest ersten Teilimplantats 102 oder des Abdichtrings 103 1/2, insbesondere 1/3, ganz besonders 1/4 der Radialkraft des zumindest zweiten Teilimplantats 104 beträgt.

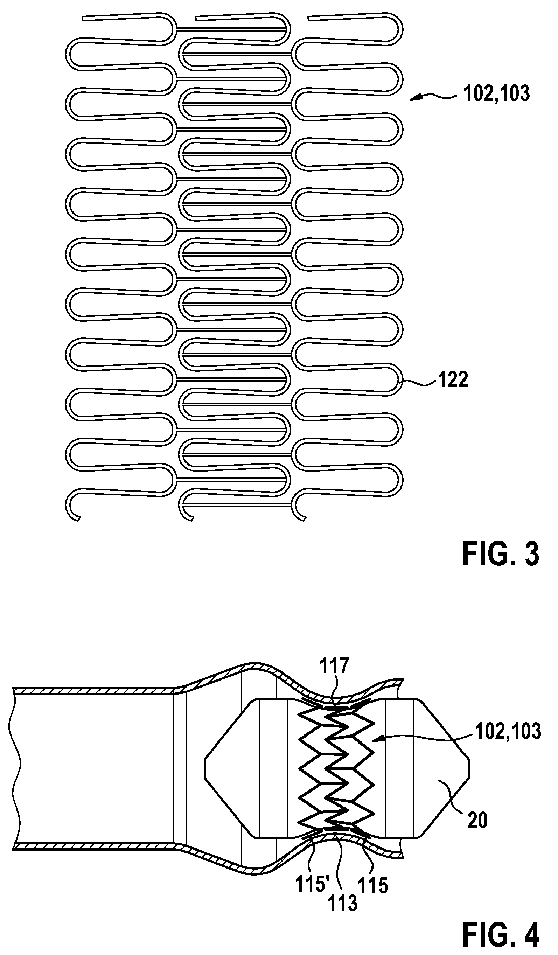

Alternativ weist das fremdexpandierbare Teilimplantat 102 oder der Abdichtring 103 ein Grundgerüst 122 auf, welche eine mäanderartige Struktur aufweist (vgl. Fig. 3 und 4). Zur besseren Übersicht wurde die quellfähige biokorrodierbare Polymermatrix 116 nicht abgebildet.

Um den Formschluss zwischen dem fremdexpandierbaren Teilimplantat 102 oder dem Abdichtring 103 zu optimieren, kann einerseits das Grundgerüst 122 in einer bevorzugten Ausgestaltung flexible Randbereiche 115, 115' und einen festeren Mittelbereich 117 mit unterschiedlichen Stegbreiten aufweisen (Fig. 4). Ein derartig aufgebautes fremdexpandierbares Teilimplantat 102 oder Abdichtring 103 ermöglicht ein verbessertes Abdichten von Leckagen am Aortenannulus 113, da durch die leicht erhöhte Radialkraft des festeren Mittelbereichs 117 im Vergleich zum flexibleren Randbereich 115, 115' die quellfähige biokorrodierbare Polymermatrix 116 gegen die Verkalkungen des gesamten Annulus 113 gedrückt werden kann.

Um den Formschluss zwischen dem fremdexpandierbaren Teilimplantat 102 oder dem Abdichtring 103 zu optimieren, kann andererseits durch eine geeignete Expansionshilfe 20 der sogenannte "Dogboning"-Effekt ausgenutzt werden. Damit wird der Effekt bezeichnet, dass sich eine mit einem aufgecrimpten Implantat versehene Expansionshilfe zuerst an den Randbereichen des Implantats öffnet. Beispielsweise kann, wie in Figur 4 gezeigt, die Expansionshilfe 20 länger seine als das fremdexpandierbare Teilimplantat 102 oder der Abdichtring 103 um somit einen Formschluss im gesamten Annulusbereich zu erzielen.

In einer besonderen Ausgestaltung der vorliegenden Erfindung ermöglicht es die Kombination eines fremdexpandierbaren Teilimplantats 102 oder eines Abdichtrings 103 mit flexiblen Randbereichen 115, 115' und festerem Mittelbereich 117 und einer Expansionshilfe 20, welche länger ist als das fremdexpandierbare Teilimplantat 102 oder der Abdichtring 103, das Anbringen der abdichtenden Polymermatrix 116 bis in die Windung des Annulus hinein. Somit ist ein herausragender Formschluss zwischen dem fremdexpandierbaren Teilimplantat 102 oder dem Abdichtring 103 mit dem Annulus 113 möglich (vgl. Fig. 4).

Das zweite Teilimplantat 104 ist bevorzugt selbstexpandierend ausgeführt und wird im folgenden Text als selbstexpandierendes Teilimplantat 104 bezeichnet (vgl. Fig. 2). Das selbstexpandierende Teilimplantat 104 ist aus einem Formgedächtnismaterial, wie beispielsweise Nitinol, gefertigt. Zudem weist das selbstexpandierende Teilimplantat 104 ein Grundgerüst 122 mit einer Anzahl von Zellen 114, 114' auf. Die Zellen 114, 114' sind im Wesentlichen rautenförmig und in Umfangsrichtung 124 nebeneinander, jeweils alternierend in der Höhe versetzt angeordnet. Ferner weist das zweite selbstexpandierende Teilimplantat 104 den Klappeneinsatz 108, beispielsweise in der Form einer künstlichen Aortenklappe aus Schweine-Perikard, auf.

### Bezugszeichenliste

- 20: Expansionshilfe
- 102: Teilimplantat
- 103: Abdichtring
- 104: Teilimplantat
- 108: Klappeneinsatz
- 113: Annulus
- 114: Zelle
- 115: Flexibler Mittelbereich
- 116: Quellfähige biodegradierbare Polymermatrix
- 117: Fester Randbereich
- 122: Grundgerüst
- 124: Umfangsrichtung

## Patentansprüche

1. Medizinisches Hybridimplantat zum Einführen mit einer Einführvorrichtung in den menschlichen oder tierischen Körper, **dadurch gekennzeichnet, dass** das Hybridimplantat zumindest zwei Teilimplantate (102, 104) aufweist, von denen das zumindest erste Teilimplantat (102) fremdexpandierbar ausgeführt wird sowie aus einem mit einer quellfähigen biodegradierbaren Polymermatrix (116) versehenen biokorrodierbaren Material besteht und das zumindest zweite Teilimplantat (104) fremdexpandierbar oder selbstexpandierbar, insbesondere selbstexpandierbar, ausgeführt wird sowie mit einem Klappeneinsatz versehen ist.

2. Medizinisches Hybridimplantat nach Anspruch 1, wobei das erste Teilimplantat (102) aus einem biokorrodierbaren Material ausgewählt aus der Gruppe der Magnesiumlegierungen besteht.

3. Medizinisches Hybridimplantat nach Anspruch 1 oder 2, wobei die Radialkraft des zumindest ersten Teilimplantats (102) ½, insbesondere 1/3, ganz besonders ¼ der Radialkraft des zumindest zweiten Teilimplantat (104) aufweist.

4. Medizinisches Hybridimplantat nach einem der vorherigen Ansprüche, wobei die quellfähige biodegradierbare Polymermatrix (116) ausgewählt ist aus der Gruppe der Hydrogele, insbesondere aus der Gruppe umfassend Hyaluronsäuren, Polysaccharide, Polyphosphazene, Cellulose, Polyehtylenglycole oder Polyacrylamid, einzeln und/oder in Kombination.

5. Medizinisches Hybridimplantat nach einem der vorherigen Ansprüche, wobei das medizinische Hybridimplantat zusätzlich ein Trennmittel, insbesondere eine Siliziumcarbid-Beschichtung und vorzugsweise aufgebracht auf dem zumindest zweiten Teilimplantat (104), aufweist.

6. Medizinisches Hybridimplantat nach einem der vorherigen Ansprüche, wobei das zweite Teilimplantat (104) aus einem Material und/oder aus einer Kombination von mehreren metallischen Materialien ausgewählt aus der Gruppe umfassend Eisen, Magnesium, Nickel, Wolfram, Titan, Zirkonium, Niob, Tantal, Zink, Silizium, Lithium, Natrium, Kalium, Kalzium, Mangan, insbesondere einer Kupfer-Zink-Aluminium-Legierung und/oder Nickel-Titan-Legierung, vorzugsweise Nitinol, besteht.

7. Einführvorrichtung zum Einführen eines medizinischen Hybridimplantats nach einem der Ansprüche 1 bis 6, wobei das Hybridimplantat zumindest zwei Teilimplantate (102, 104) aufweist, von denen zumindest ein erstes Teilimplantat (102) fremdexpandierbar und zumindest ein zweites Teilimplantat (104) selbstexpandierend ausgeführt ist.

8. Implantierbarer Abdichtring 103 zum Aufnehmen eines mit einem Klappeneinsatz versehenen Teilimplantats (104), **dadurch gekennzeichnet, dass** der Abdichtring als Teilimplantat (102) gemäß der Ansprüche 1 bis 4 ausgeführt wird.

9. Verfahren zu einem Einführen eines medizinischen Hybridimplantats, insbesondere nach zumindest einem der Ansprüche 1 bis 6, mit Hilfe einer Einführvorrichtung, insbesondere nach einem der Ansprüche 7.
